(19) Europäisches Patentamt
European Patent Office
Office européen des brevets

(11) Publication number: 0 167 376 B1

# (12) EUROPEAN PATENT SPECIFICATION

(45) Date of publication of patent specification: **15.03.89**

(51) Int. Cl.⁴: **C 07 D 307/58,** A 23 L 1/226, C 11 B 9/00

(21) Application number: **85304674.6**

(22) Date of filing: **01.07.85**

## (54) Pineapple ketone carbonate derivatives.

(30) Priority: **02.07.84 US 627311**

(43) Date of publication of application:
**08.01.86 Bulletin 86/02**

(45) Publication of the grant of the patent:
**15.03.89 Bulletin 89/11**

(84) Designated Contracting States:
**AT BE CH DE FR GB IT LI NL SE**

(56) References cited:
**FR-A- 211 457**
**FR-A-2 118 954**
**GB-A-1 476 711**
**US-A-4 234 616**
**US-A-4 397 789**

(73) Proprietor: **HERCULES INCORPORATED**
**Hercules Plaza**
**Wilmington Delaware 19894 (US)**

(72) Inventor: **Byrne, Brian**
**84 Branch Brook Drive**
**Belleville New Jersey 07109 (US)**
Inventor: **Lawter, Louise Marie Lafleur**
**35 Glen Drive**
**Goshen New York 10424 (US)**

(74) Representative: **De Minvielle-Devaux, Ian Benedict Peter et al**
**CARPMAELS & RANSFORD 43, Bloomsbury Square**
**London WC1A 2RA (GB)**

**EP 0 167 376 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to flavors and specifically to modified pineapple ketone.

Pineapple ketone is the common name for the chemical 2,5-dimethyl-4-hydroxy-3(2H)-furanone. This is a compound found in pineapples, strawberries, raspberries, meats, and other foods. It has been found in cooked, roasted and fermented foods including coffee, roasted filbert, roasted almond and soy sauce. Pineapple ketone is known to be formed by the non-enzymatic browning process that occurs during roasting and baking.

Because of its cotton-candy, carmelized-sugar flavor, pineapple ketone is used extensively to compound synthetic flavors. Pineapple ketone reacts readily with amines, aldehydes and oxygen. In such cases, the pineapple ketone content of the flavors is reduced, lowering the effectiveness of the flavor.

When pineapple ketone is used in chewing-gum, it is quickly "washed out" by the chewing process, resulting in rapid loss of flavor. This "washing out" effect is due to pineapple ketone being water soluble.

Willhalm, et al., U.S. Patent No. 3,455,702; Herman, et al., U.S. Patent No. 3,697,291; and Demole, U.S. Patent No. 3,983,885 disclose that flavor may be imparted to foodstuffs, beverages, meat, or tobacco by incorporating therein a minor proportion of a dihydrofuran.

Bruns, et al., U.S. Patent No. 4,033,993; Grubbs, et al., U.S. Patent 4,127,601; and Boden, et al., U.S. Patent 4,397,789 each disclose carbonates used for their aroma. GB—A—1416711 describes 2,5-dimethyl-4-methoxy-3(2H)furanone as an active ingredient of perfume compositions but does not mention any advantageous properties of this compound compared with pineapple ketone.

According to the invention a new pineapple ketone carbonate compound is provided having the general formula,

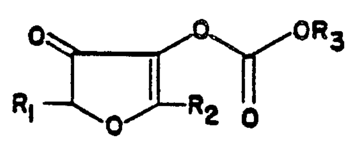

wherein $R_1$ and $R_2$ independently are $-H$, $-CH_3$, or $-CH_2CH_3$; and $R_3$ is alkyl of from 1 to 10 carbons or aryl of from 6 to 10 carbons.

The invention solves the wash out problems associated with the use of pineapple ketone, while extending the flavor over a longer period of time. By reacting pineapple ketone with an ethyl chloroformate or other alkyl chloroformates in triethylamine, the new and improved pineapple ketone carbonates are formed.

The invention relates to new carbonates derived from pineapple ketone. By forming the new carbonate derivatives, the chemical reactivity of the resulting compounds is unexpectedly reduced compared with pineapple ketone; however, the flavor of the derivatives remains very similar to the flavor of pineapple ketone. This allows direct replacement of new carbonate derivatives in applications where pineapple ketone is normally used. Additionally, the increased oxidative stability of these derivatives over pineapple ketone allow their use in applications where pineapple ketone cannot be used or does not perform well. Such applications include but are not limited to perfumes, dry flavors and tobacco.

Furthermore, the new carbonate derivatives are not as water soluble as pineapple ketone, which allows these derivatives to be preferentially dissolved by chewing- or bubble-gum-base. This effect allows the derivatives to liberate pineapple ketone-like flavor slowly during the duration of the chew, creating a flavor prolongation effect.

The method of making new carbonates are novel because, although alcohols are known to react with ethyl chloroformate and other alkyl chloroformates to form alkyl carbonate derivatives, ketones or diones are not known to react with alkyl chloroformates to form carbonate derivatives.

An unexpected aspect of the invention is that the subject derivatives are more stable to oxidation than pineapple ketone. Hirvi, et al. [Lebensm.-Wiss. u.-Technol., 13, 324 (1980)] have indicated how sensitive pineapple ketone is to oxidation. At pH 4 it has a half-life of 120 days, while at pH 7 it has a half-life of 12 days. The increased oxidative stability of the subject carbonate derivatives over pineapple ketone is given in Example 3. An unexpected aspect of this invention is that the derivatives have a similar taste to pineapple ketone.

The invention offers the following advantages:

a) The new carbonates are flavor properties similar to pineapple ketone;

b) The new carbonates offer oxidative stabilities superior to pineapple ketone, allowing them to be used in situations where pineapple ketone will be unstable. Such situations include use as liquid flavors, and especially dry flavors;

c) The new carbonates can be used in perfumes, whereas pineapple ketone is unstable leading to changes in the fragrance profile over time and discoloration of the formulation;

d) The new carbonates prolong flavor in chewing-gum and bubble-gum.

Exemplary of the preferred extended flavor compounds and the invention are:

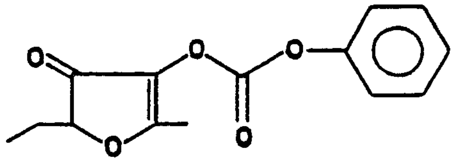
;

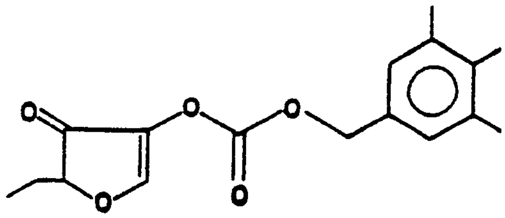
;

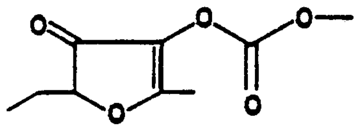
;

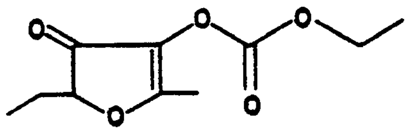
;

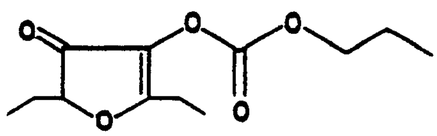
;

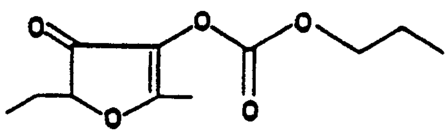
;

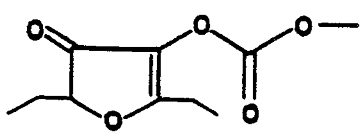
;

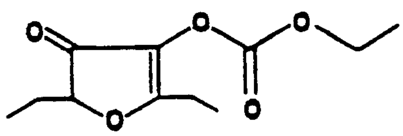
;

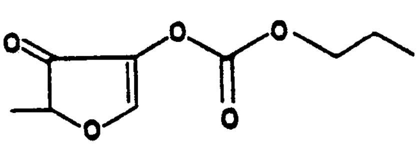
;

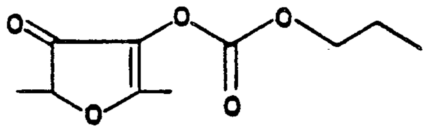

;

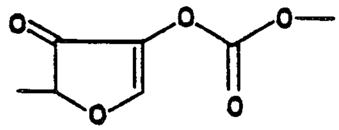

;

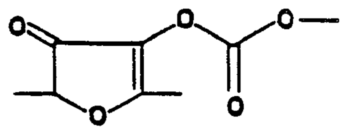

;

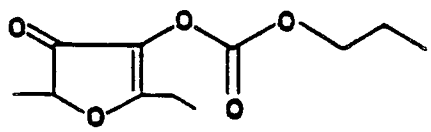

;

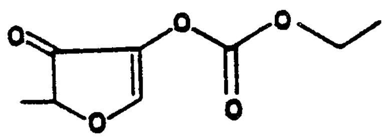

;

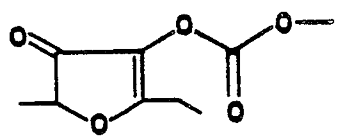

;

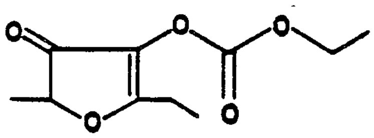

;

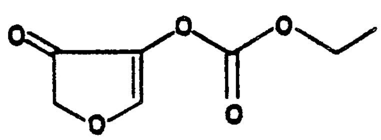

;

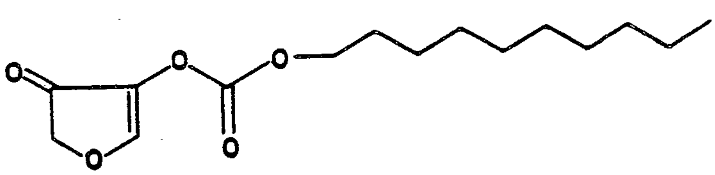

;

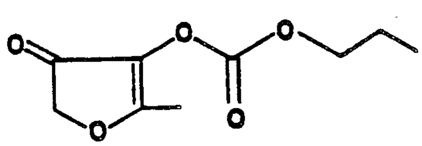

; and

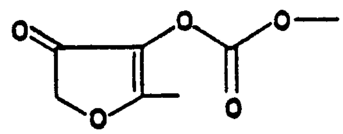

Example 1

*Preparation of ethyl 2,5-dimethyl-3-oxo-4(2H)-furyl carbonate*

A solution of 2,5-dimethyl-4-hydroxy-3(2H)-furanone (pineapple ketone, 133.0g, 1.0 mole) and triethylamine (152.0g, 1.5 moles) in methylene chloride (665g) is cooled to 5°C under nitrogen atomosphere. To the cooled solution is added over a period of 2.5 hours, a solution of ethyl chloroformate (127.7g, 1.18 moles) in methylene chloride (600g) stirred at 5—8°C for two additional hours. The reaction mixture is filtered through a Buchner funnel to remove the triethylamine hydrochloride. The salt is washed with methylene chloride (200 ml). The combined organic layers are washed twice with 100 ml portions of water and dried over sodium sulfate. The solvent is removed in vacuo and the crude product distilled (0.1 torr) through a Goodloe column to yield 72.5% of ethyl 2,5-dimethyl-3-oxo-4(2H)-furyl carbonate product b.p. 87°C (0.08 torr), IR (neat film) 2980(M), 1770(S), 1715(S), 1640(S), 1250(S) $cm^{-1}$, 'H NMR 1.58(t,J = 8Hz,3), 1.65 (d,J = 7Hz,3), 2.32 (s,3), 4,45 (q,J = 7Hz,2), 4.75 (q,J = 7Hz,1), and 3.3% of the diadduct, diethyl 2,5-dimethyl-3,4-furyl dicarbonate, b.p. 110°C (0.08 torr), IR (neat film 2980(M), 1770(S), 1250(S), 'H NMR = 1.50 (t,J = 6Hz,6) 2.3 (S,6) 4.45 (q,J = 8Hz,4).

Example 2

*Preparation of methyl 2,5-dimethyl-3-oxo-4(2H)-furyl carbonate*

A solution of 2,5-dimethyl-4-hydroxy-3(2H)-furanone (pineapple ketone, 44.0g, 0.33 mole) and triethylamine (50.5g, 0.5 mole) in methylene chloride (220g) is cooled to 5°C under nitrogen atmosphere. To the cooled solution is added over a period of 2.0 hours, a solution of methyl chloroformate (38.6g, 0.41 mole) in methylene chloride (200g), which is then stirred at 5—8°C for two additional hours. The reaction mixture is filtered through a Buchner funnel to remove the triethylamine hydrochloride. The salt is washed with methylene chloride (200 ml). The combined organic layers are washed twice with 100 ml portions of water and dried over sodium sulfate. The solvent is removed in vacuo and the crude produce distilled (0.1 torr) through a Goodloe column to yield 18g or methyl 2,5-dimethyl-3-oxo-2(2H)-furyl carbonate product, b.p. 105°C; IR (neat film) 2960(M), 1770(S), 1710(S), 1640(S), 1250(S), 1200(S) $cm^{-1}$; 'H NMR 1.55 (d, J = 8Hz,3), 2.28 (s,3), 3.95 (s,3), 4.70 (q, J = 8Hz,1).

Example 3

*Oxidative stability of ethyl 2,5-dimethyl-3-oxo-4(2H)-furyl carbonate*

Solutions containing respectively 10 percent carbonate and ten percent pineapple ketone in toluene are stirred in loosely capped vials at ambient temperature. Samples are taken periodically and the concentration of carbonate or ketone remaining determined by GLC (6′ × ⅛″, 15% Carbowax-20M on 80/100 Chromasorb W, Helium = 28 cc/min, 100°—210° at 8°/min). The experiment is repeated using ethanol as solvent. Results are shown below.

COMPARATIVE STABILITY IN AIR
(Toluene, Ambient Temperature)

| PINEAPPLE KETONE | | ETHYL CARBONATE OF EXAMPLE 1 | |
|---|---|---|---|
| Time (hours) | % Remaining | Time (hours) | % Remaining |
| 0 | 100 | 0 | 100 |
| 15 | 24 | 17 | 100 |
| 41 | 18 | 40 | 100 |
| 136 | 6 | 117 | 100 |
| 138 | 4 | 137 | 100 |

COMPARATIVE STABILITY IN AIR
(Ethanol, Ambient Temperature)

| PINEAPPLE KETONE | | ETHYL CARBONATE OF EXAMPLE 1 | |
|---|---|---|---|
| Time (hours) | % Remaining | Time (hours) | % Remaining |
| 0 | 100 | 0 | 100 |
| 21 | 100 | 21 | 100 |
| 48 | 58 | 46 | 100 |
| 72 | 30 | 69 | 100 |
| 149 | 8 | 149 | 100 |
| 172 | 9 | 173 | 100 |

Example 4
*Bubble Gum*

A typical bubble gum base is made with the following ingredients:

| | |
|---|---|
| Bubble Base T, Acid Balanced (L. A. Dreyfus Co., South Plainfield, NJ) | 135.00 parts |
| Corn syrup, 43° baume | 161.25 parts |
| Powdered sugar, confectioners 10X | 450.00 parts |
| Citric acid | 3.75 parts |
| Glycerine | 3.75 parts |
| Flavors | 7.50 parts |

All ingredients are mixed in a gum blender with a jacketed sidewall. To gum A is added 7.50 parts strawberry flavor 500389—U (Hercules, PFW Division, Middletown, NY). To gum B is added 7.125 parts strawberry flavor 500389—U, plus 0.375 parts of ethyl carbonate pineapple ketone derivative from Example 1. The gums are cut into 5.0 grams pieces and evaluated by panelists. Both gums A and B have a long-lived strawberry flavor, with gum B having a higher overall rating for flavor prolongation after 10 minutes, as well as, having a higher and more sustained flavor intensity peak during the middle of the chew. Gum B has the best retained strawberry character throughout the chew.

**Claims for the Contracting States: BE CH DE FR GB IT LI NL SE**

1. A compound of the general formula,

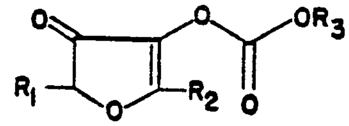

wherein $R_1$ and $R_2$ independently are $-H$, $-CH_3$, or $-CH_2CH_3$; and $R_3$ is alkyl of from 1 to 10 carbons or aryl of from 6 to 10 carbons.

2. The compound of claim 1 having the formulas:

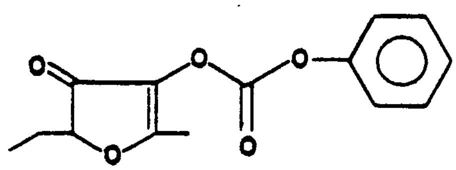

or

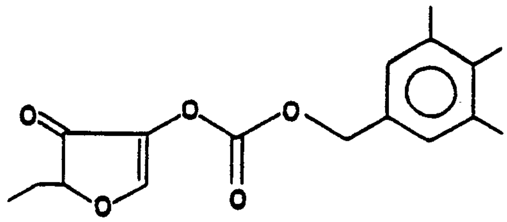

3. The compound of claim 1 having the formulas:

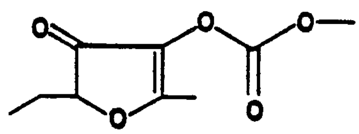

,

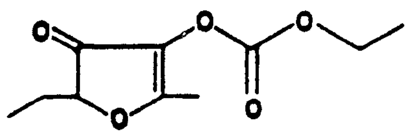

or

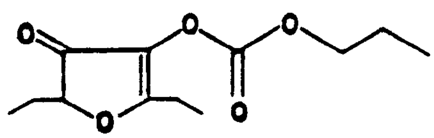

.

4. The compound of claim 1 having the formulas:

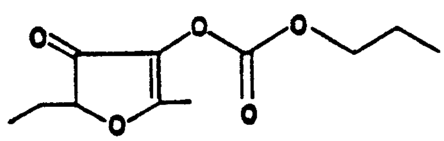

,

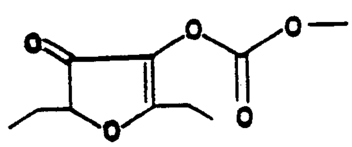

,

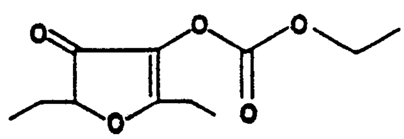

or

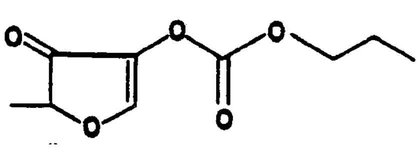

.

5. The compound of claim 1 having the formulas:

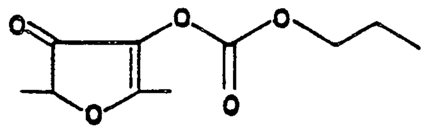

,

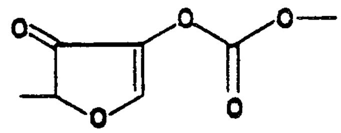

,

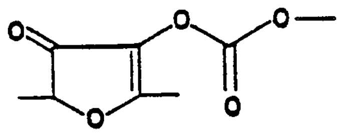

or

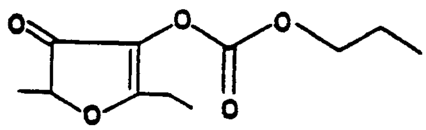

6. The compound of claim 1 having the formulas:

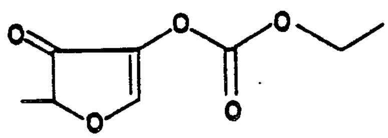

,

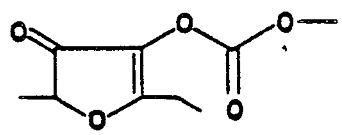

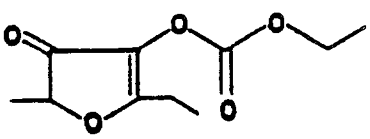

or

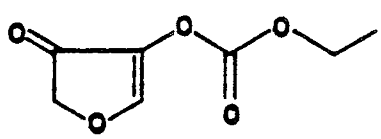

.

7. The compound of claim 1 having the formulas:

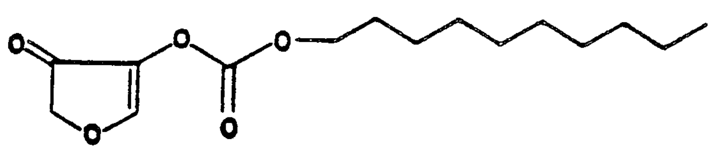

;

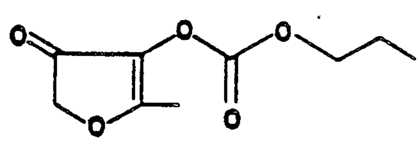

;

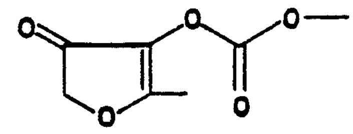

or

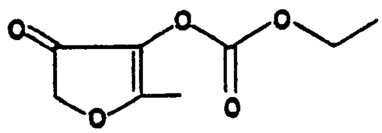

.

8. The compound of claim 1 having the formulas:

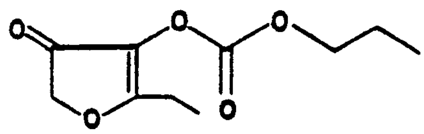

,

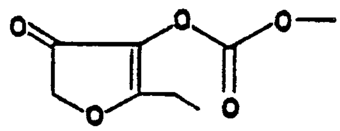

,

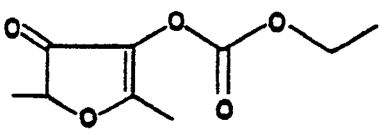

or

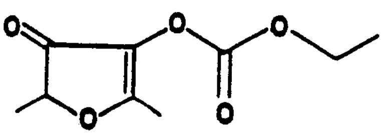

.

9. The compound of claim 1 in combination with a perfume and/or a fragranced composition.

10. The compound of claim 1 in combination with a flavor and/or foodstuff.

11. The composition of claim 10 wherein said foodstuff is chewing gum or bubble gum.

12. The compound of claim 1 in combination with toothpaste or tobacco.

**Claims for the Contracting State: AT**

1. A process for the production of a pineapple ketone carbonate compound of the general formula

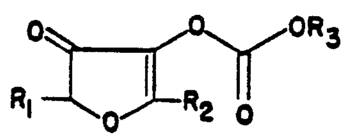

wherein $R_1$ and $R_2$ independently are $-H$, $-CH_3$, or $-CH_2CH_3$; and $R_3$ is alkyl of from 1 to 10 carbons or aryl of from 6 to 10 carbons, which comprises reacting pineapple ketone with an appropriate chloroformate.

2. A process according to claim 1 in which the said reaction is carried out in triethylamine.

**Patentansprüche für die Vertragsstaaten: BE CH DE FR GB IT LI NL SE**

1. Eine Verbindung der allgemeinen Formel

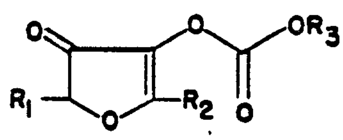

wobei $R_1$ und $R_2$ jeweils $-H$, $-CH_3$ oder $-CH_2CH_3$ sind und $R_3$ eine Alkylgruppe mit 1 bis 10 C—Atomen oder eine Arylgruppe mit 6 bis 10 C—Atomen ist.

2. Verbindung nach Anspruch 1, dadurch gekennzeichnet, daß sie die Formeln

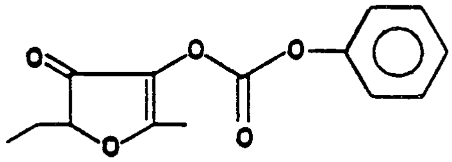

oder

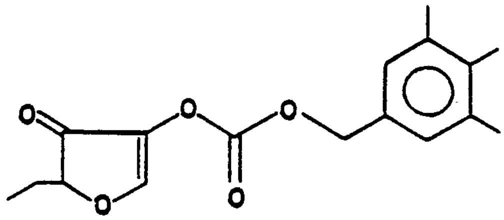

besitzt.

3. Verbindung nach Anspruch 1, dadurch gekennzeichnet, daß sie die Formeln

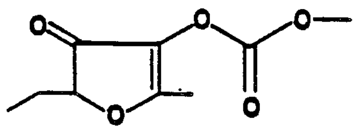

,

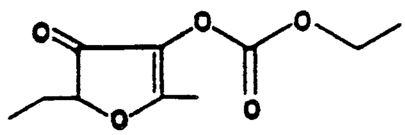

oder

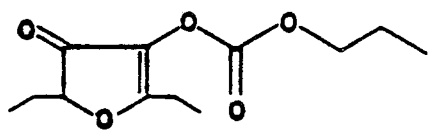

besitzt.

4. Verbindung nach Anspruch 1, dadurch gekennzeichnet, daß sie die Formeln

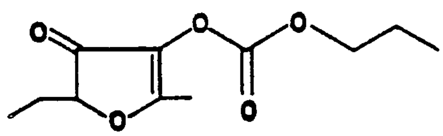

,

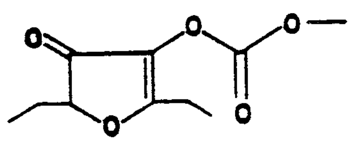

,

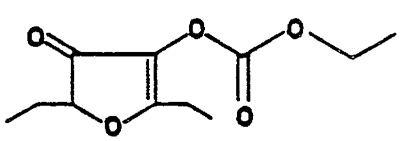

oder

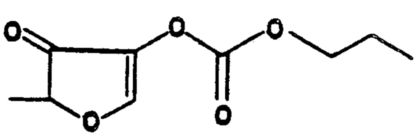

besitzt.

5. Verbindung nach Anspruch 1, dadurch gekennzeichnet, daß sie die Formeln

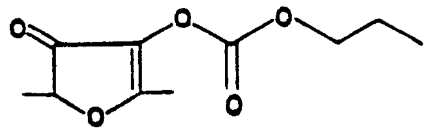

,

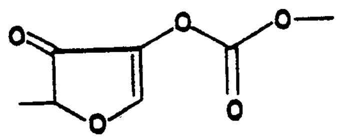

,

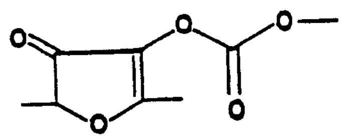

oder

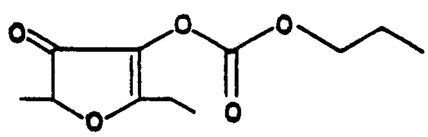

besitzt.

6. Verbindung nach Anspruch 1, dadurch gekennzeichnet, daß sie die Formeln

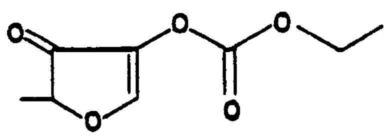

,

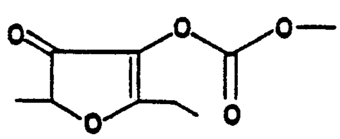

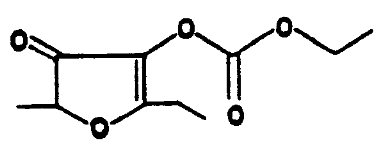

oder

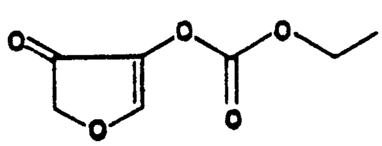

besitzt.

7. Verbindung nach Anspruch 1, dadurch gekennzeichnet, daß sie die Formeln

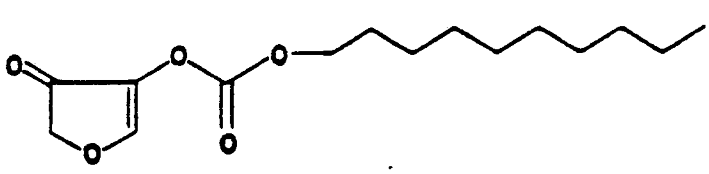

,

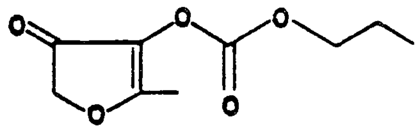

,

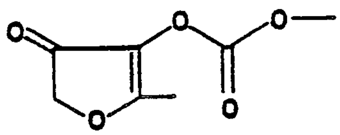

oder

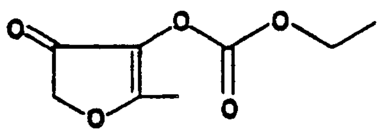

besitzt.

8. Verbindung nach Anspruch 1, dadurch gekennzeichnet, daß sie die Formeln

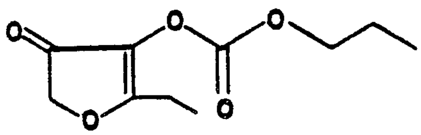

,

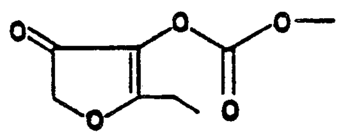

,

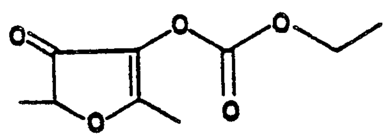

oder

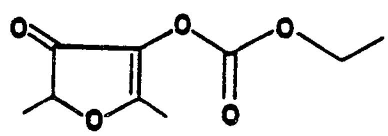

besitzt.

9. Verbindung nach Anspruch 1, in Verbindung mit einem Parfum und/oder einer Duftzusammensetzung.

10. Verbindung nach Anspruch 1, in Verbindung mit einem Geschmackstoff und/oder einem Lebensmittel.

11. Zusammensetzung nach Anspruch 10, wobei das genannte Lebensmittel Kaugummi oder Blaskaugummi ist.

12. Verbindung nach Anspruch 1 in Verbindung mit Zahnpaste oder Tabak.

**Patentansprüche für den Vertragsstaat: AT**

1. Verfahren zur Herstellung einer Ananasketonkarbonatverbindung der allgemeinen Formel

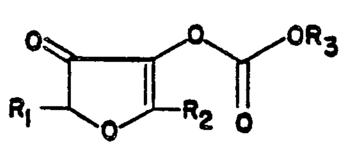

,

worin $R_1$ und $R_2$ jeweils $-H$, $-CH_3$ oder $-CH_2CH_3$ sind und $R_3$ eine Alkylgruppe mit 1 bis 10 C-Atomen oder eine Arylgruppe mit 6 bis 10 C-Atomen ist, dadurch gekennzeichnet, daß Ananasketon mit einem geeigneten Chloroformat umgesetzt wird.

2. Verfahren nach Anspruch 1, in welchem die genannte Reaktion in Triethylamin ausgeführt wird.

**Revendications pour les Etats Contractants: BE CH DE FR GB IT LI NL SE:**

1. Composé de formule générale,

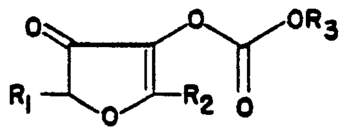

dans laquelle $R_1$ et $R_2$ sont, indépendamment l'un de l'autre, —H, $—CH_3$, ou $—CH_2CH_3$ et $R_3$ est un radical alkyle comprenant de 1 à 10 atomes de carbone ou un radical aryle comprenant de 6 à 10 atomes de carbone.

2. Composé suivant la revendication 1 ayant les formules:

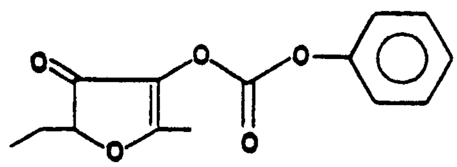

ou

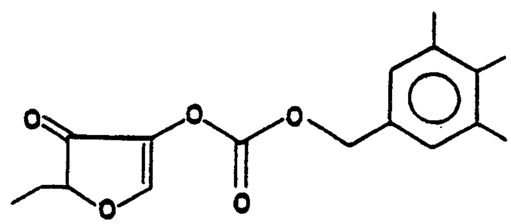

3. Composé suivant la revendication 1 ayant les formules:

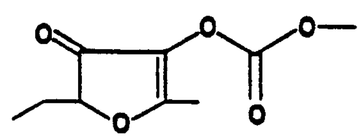

,

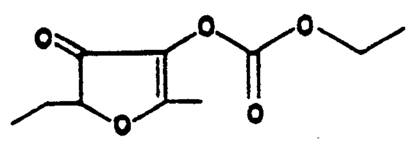

ou

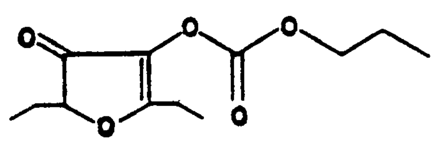

.

4. Composé suivant la revendication 1 ayant les formules:

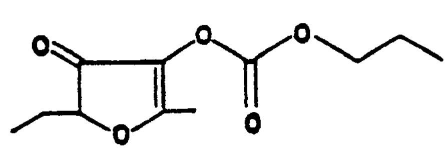

,

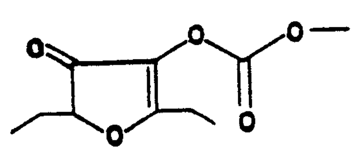

,

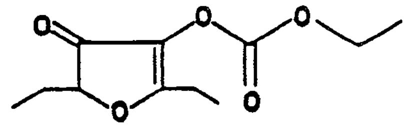

ou

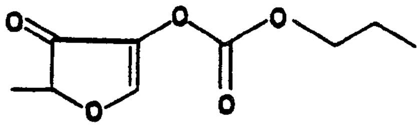

.

5. Composé suivant la revendication 1 ayant les formules:

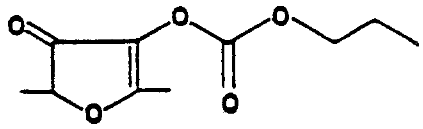

,

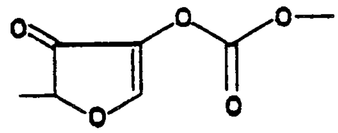

,

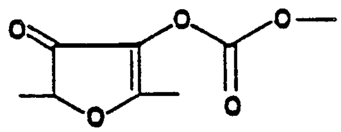

ou

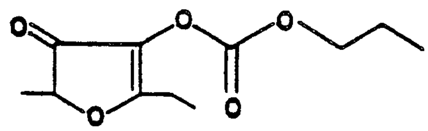

6. Composé suivant la revendication 1 ayant les formules:

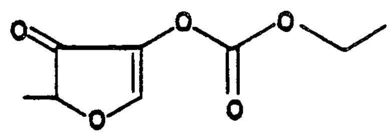

,

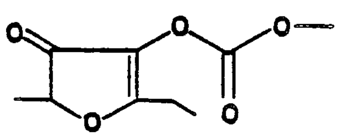

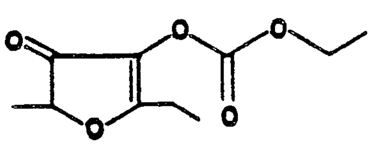

ou

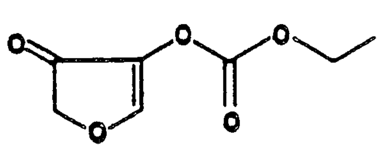

.

7. Composé suivant la revendication 1 ayant les formules:

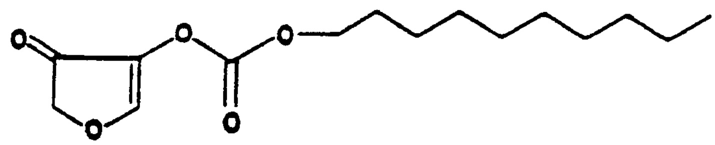

;

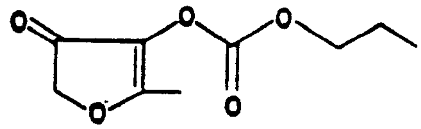

;

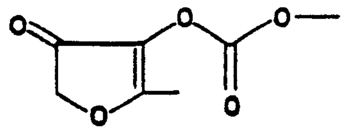

ou

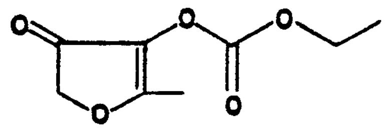

.

8. Composé suivant la revendication 1 ayant les formules:

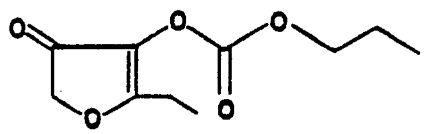

,

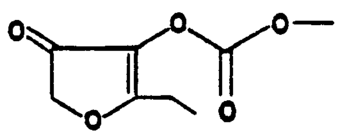

,

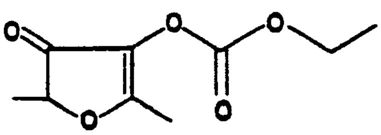

ou

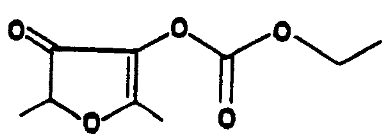

.

9. Composé suivant la revendication 1 en combinaison avec un parfum et/ou composition perfumée.

10. Composé suivant la revendication 1 en combinaison avec un arôme et/ou un aliment.

11. Composé suivant la revendication 10 dans laquelle l'aliment est un chewing gum ou une gomme à bulles.

12. Composé suivant la revendication 1 en combinaison avec un dentifrice ou un tabac.

**Revendications pour l'Etate contractant: AT**

1. Procédé de fabrication d'un composé de carbonate de cétone d'ananas ayant la formule générale,

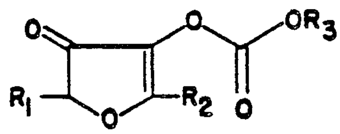

dans laquelle $R_1$ et $R_2$ sont, indépendamment l'un de l'autre, —H, —$CH_3$, ou —$CH_2CH_3$ et $R_3$ est un radical alkyle comprenant de 1 à 10 atomes de carbone ou un radical aryle comprenant de 6 à 10 atomes de carbone, caractérisé en ce qu'on fait réagir une cétone d'ananas avec un chloroformale approprié.

2. Procédé suivant revendication 1 caractérisé en ce que la réaction est effectuée dans de la triéthylamine.